(19) Europäisches Patentamt
European Patent Office
Office européen des brevets

(11) EP 0 749 575 B1

(12) **EUROPEAN PATENT SPECIFICATION**

(45) Date of publication and mention
of the grant of the patent:
**15.10.1997 Bulletin 1997/42**

(51) Int Cl.6: **G01N 23/00**, G01N 33/02,
G01N 33/24

(21) Application number: **95912144.3**

(22) Date of filing: **09.03.1995**

(86) International application number:
**PCT/DK95/00110**

(87) International publication number:
**WO 95/24637 (14.09.1995 Gazette 1995/39)**

(54) **A METHOD OF DETECTING EARTH IN AGRICULTURAL CROPS**

EIN VERFAHREN ZUM NACHWEIS VON ERDE IN LANDBAUPRODUKTEN

PROCEDE DE DETECTION DE LA PRESENCE DE TERRE DANS LES RECOLTES AGRICOLES

(84) Designated Contracting States:
**AT BE CH DE ES FR GB GR IE IT LI NL PT SE**

(30) Priority: **09.03.1994 DK 272/94**

(43) Date of publication of application:
**27.12.1996 Bulletin 1996/52**

(73) Proprietor: **DANISCO A/S**
**1411 Copenhagen K. (DK)**

(72) Inventors:
• **J RGENSEN, Lars, Bo**
**DK-4800 Nyk bing F (DK)**
• **FRYDENDAL, Ib**
**DK-4983 Dannemare (DK)**
• **HANSEN, Ole**
**DK-4983 Dannemare (DK)**

(74) Representative: **Siiger, Joergen et al**
**c/o Chas. Hude**
**H.C. Andersens Boulevard 33**
**1553 Copenhagen V (DK)**

(56) References cited:
**EP-A- 0 459 648**          **WO-A-94/17404**
**US-A- 5 003 490**

• **Proceedings of the 14th Conference 1992 of the Australian Society of Sugar Cane Technologists held at Mackay, Queensland from 28th April to 1st May 1992, P.G. Atherton et al: "The Determination of Soil in Cane"**

**Description**

Technical Field

The invention relates to a method of detection the contents of earth in agricultural crops, such as beets, by measuring the natural $y$-radiation of the earth, the counting number of the measured $y$-radiation being compensated for differences in types of earth by way of an analysis of the $y$-spectrum.

Background Art

It is known that it is possible to reduce the degree of inaccuracy by compensating for the humus contents of the earth, see for example P.G. Atherton et al: "The Determination of Soil in Cane", Proc. of the 14th conf. 1992 of the Australian Soc. of Sugar Techn., Mackay, 1992.

Brief Description of the Invention

The object of the invention is to provide a method of further reducing the degree of inaccuracy.

A method of the above type is according to the invention characterised in that the $y$-spectrum is analyzed by means of a neural network with a high number of inputs, where a signal representing the counting number in a separate energy range of the $y$-radiation is fed to each input, said neural network in advance being programmed by means of known references. In this manner many of the data contained in the spectrum are utilized, and it turned out that the degree of inaccuracy can be almost halved.

According to a particularly advantageous embodiment of the method the neural network may according to the invention comprise 14 inputs, 13 inputs for the insertion of the counting number representing 13 different windows and one input for the insertion of the total counting number referred to as the integral counting number.

Brief Description of the Drawings

The invention is explained in greater detail below with reference to the accompanying drawings, in which

Fig. 1 illustrates a spectrum of the natural $y$-radiation from earth on beets,

Fig. 2 illustrates a mathematical function forming part of a neural network in form of a non-linear element for earth type correction,

Fig. 3 illustrates a first embodiment of a neural network with one output,

Fig. 4 illustrates a second embodiment of a neural network with two outputs,

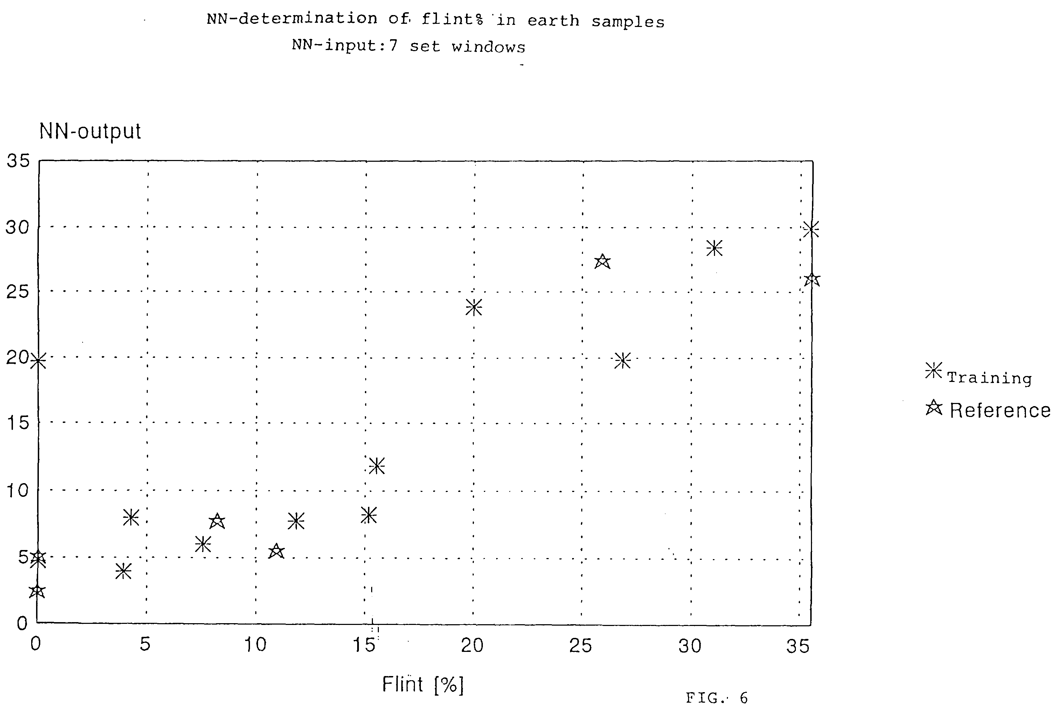

Figs. 5 and 6 are views verifying that it is possible to compensate for the contents of water and flint, respectively, in the earth.

Best Mode for Carrying Out the Invention

In order to reduce the number of inputs for a neural network for analyzing spectra of natural $y$-radiation the previously achieved results are utilized, said results being obtained from the spectra together with statistic analyses for splitting up said spectra and being utilized such that a minimum of parameters provide the maximum number of data. The $y$-spectra included originally 1024 counting numbers representing energies from 0 to 3.07 MeV and were scanned in 13 windows with the following channel numbers:

1:          75          -          100

| | | | |
|---|---|---|---|
| 2: | 101 | - | 125 |
| 3: | 126 | - | 147 |
| 4: | 148 | - | 175 |
| 5: | 176 | - | 200 |
| 6: | 201 | - | 240 |
| 7: | 241 | - | 270 |
| 8: | 271 | - | 295 |
| 9: | 296 | - | 360 |
| 10: | 361 | - | 410 |
| 11: | 411 | - | 450 |
| 12: | 451 | - | 530 |
| 13: | 550 | - | 900 |

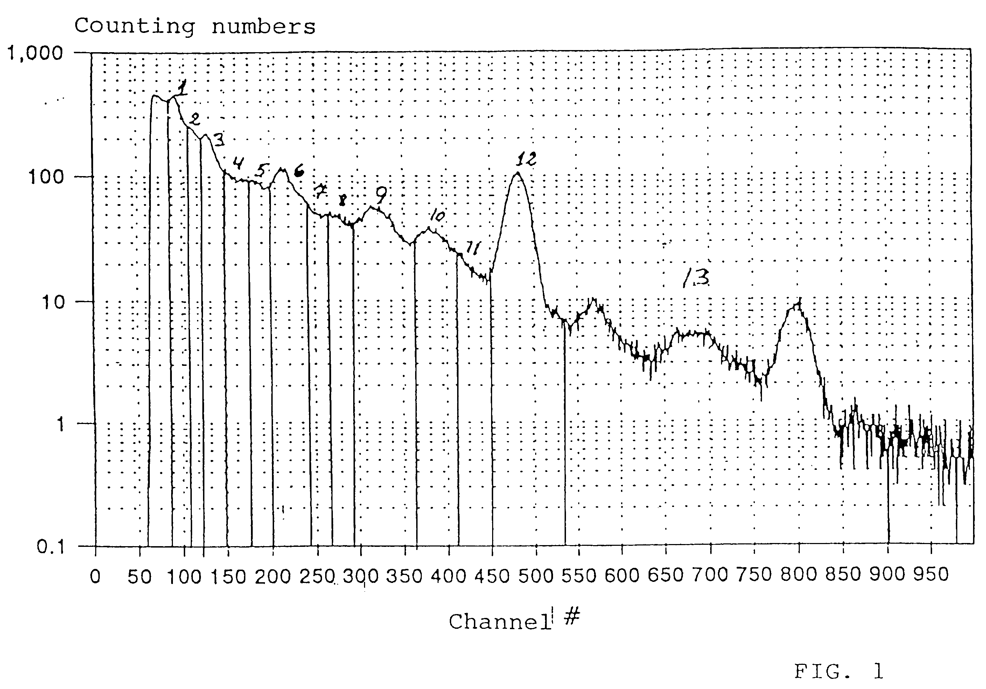

Channel number 487 represents the centre of the potassium peak (1.46 MeV). A typical spectrum with the windows indicated therein appears from Fig. 1.

The parts of the spectrum below channel 75 and above channel 900 are not used.

The countings including 13 counting numbers $S_1$, $S_2$, .....$S_{13}$ are set such that the sum is 1.0, i.e.

$$\overline{S} = (s_1, s_2, ..., s_{13}); \qquad \Sigma s_i = 1,0$$

Each of the 13 counting numbers $S_1$, $S_2$, .....$S_{13}$ is divided with a number during the setting, said number being the integral counting number Intg of the spectrum in question. The integral counting number is stored together with each set of counting numbers. The sets of counting numbers are used for training a neural network for earth type correction. The normalized counting numbers are used as input.

Attempts have been made at simulating the content of potassium in beets by means of a set of data with addition of potassium in connection with a measuring of earth in loads of beets. The previous counting numbers $S_1$, $S_2$.....$S_{13}$ are used together with a corresponding set of data of pure potassium K for generating a new set of data,

$$\overline{A}_\alpha = \frac{\overline{M}_\alpha}{\Sigma m_i}$$

and the set of data $M_\alpha$ results from

$$\overline{M}_\alpha = \overline{S} \times Intg + \overline{K} \times 40000 \times \alpha = (m_1, m_2, ..., m_{13})_\alpha$$

and $\alpha \in \{0.0; 0.1; 0.2; 0.3; 0.4; 0.5; 0.6\}$. A constant of 40,000 is used because the average number of the integral counting numbers is of this value. Thus a kind of analogy exists from for instance $\alpha = 0.3$ to the fact that a quantity is added to the original $y$-spectrum, said quantity in the integral counting number meaning that 30% extra is added in form of potassium.

Now 7 sets of data are generated from the original sets of data. The 13 normalized counting numbers are again used as input; the only desired output is the integral counting number Intg whereas the other output is $\alpha$ times 40,000.

2/3 of the sets of data are used as sets for training while the last third is used for testing the network during a training of the network. When a network is programmed to nothing but earth type correction, 70 sets of data are used for the training while the remaining 34 sets of data are used for testing of the network.

Below the structure of a neuron in a neural feed forward network for earth type correction is explained in greater detail.

A neuron can be considered as a function with an arbitrary number of inputs (independent variables) and only one output (depending on the inputs). A weight factor $w_k$ corresponds to the k'th input $I_k$ matches . For each input the product $I_k w_k$ is calculated, and for the neuron as a unity the output

$$O = \sigma(\sum_{k=1}^{N} I_k w_k - \theta)$$

is calculated, where the sigmoid-function

$$\sigma(x) = \frac{2}{1-e^{-x}} - 1$$

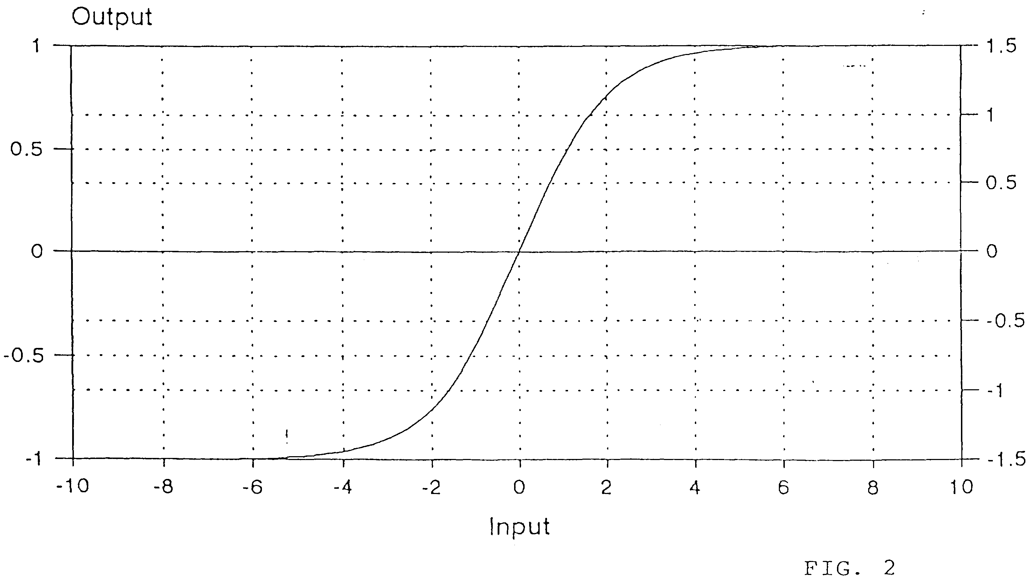

and
$\theta$ is a threshold value associated with the neuron. The $\sigma$-function is monotonously increasing and is shown in Fig. 2. It should be noted that the weight factors are associated with the neuron in question. The remaining neurons are associated with their own weight factors.

In order to simplify the training, an O'th input, $I_o$, is introduced, said input being -1. When the corresponding O'th weight factor is added to

$$w_o = \theta,$$

being the threshold value of the neuron, the expression of the output is simplified into

$$O = \sigma(\sum_{k=1}^{N} I_k w_k - \theta) = \sigma(\sum_{k=0}^{N} I_k w_k)$$

The function used for $\sigma$ is not decisive. It need only be differential and non-linear. The training used does not involve a requirement to the function being increasing across the entire interval. A sinus function can for instance also be involved. As an alternative, the function can be of the type tanh(x).

The neural network includes a perceptron with six hidden neurons and one 1 output-neuron.

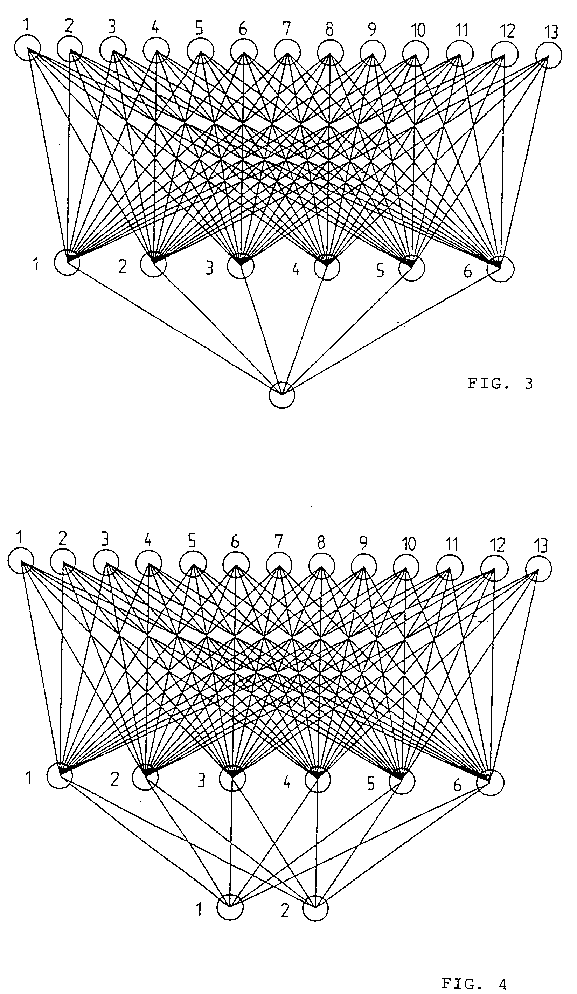

The network is shown in Fig. 3, and it appears that the 13 inputs are not internally connected, and that the hidden neurons are not internally connected either. The network is a feed-forward network.

The output from an arbitrary neuron is calculated on the basis of all inputs for the neuron (+ the O'th = -1), the 13 weight factors and the threshold value:

$$O_j = \sigma(\sum_{k=0}^{N} I_k w_{jk})$$

When the output from the latest hidden layer of neurons has been calculated, the final output of the perceptron is calculated by:

$$O = \sigma(\sum_{j=0}^{M} O_j v_j)$$

where the j'th weight factor in the 2nd layer is designated $v_j$, and where the output of the hidden neurons is the input to the outermost neuron. The output is calculated by means of the sigmoid-function. This function can, however, be omitted. When it is omitted, the output-neuron allows a scaling of the output to an arbitrary interval.

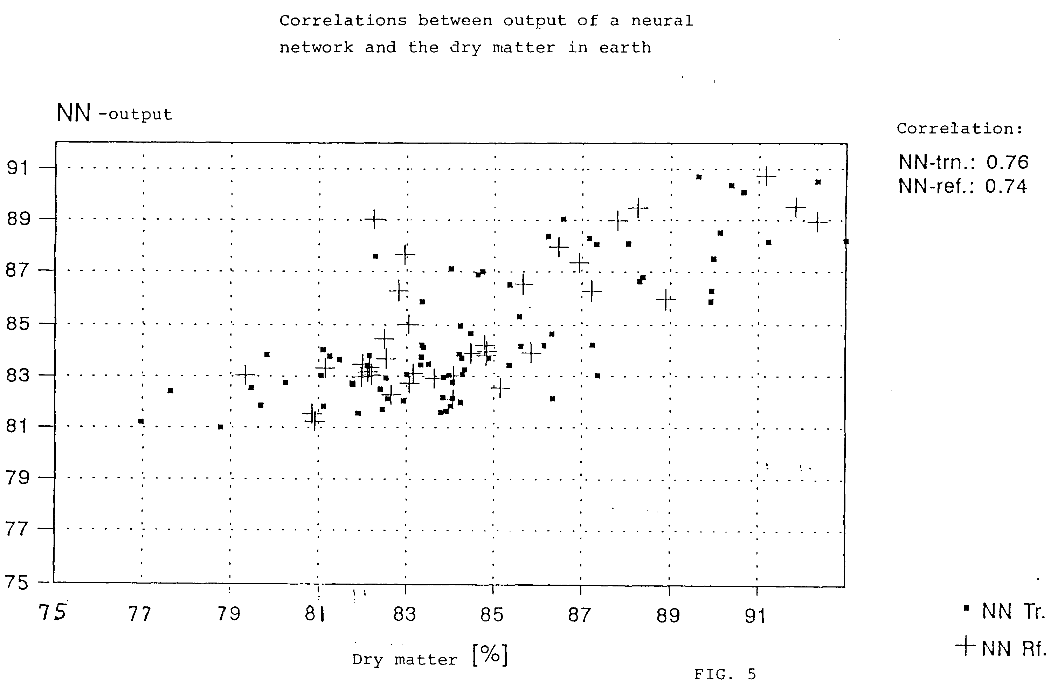

The above described perceptron comprises one output. Nothing prevents, however, this perceptron from comprising more outputs. One perceptron with two outputs is used for the part of the training where the set of data derives from samples containing potassium. Then one of the two outputs of the perceptron indicates the integral counting number, while the other output indicates the integral counting number for potassium. The network is shown in Fig. 4. The difference is due to the fact that one set of weight factors and threshold values exists for each of the two outputs. The calculations are the same as previously. In the network in Fig. 4, the perceptron includes, however, 8 hidden neurons instead of 6.

The most ordinary method of training a feed-forward network is the "error back propagation"-method. According to the invention, a simplified method has, however, been developed, viz. the Newton-Raphson-Frydendal-method, which is referred to below as the NRF-method.

The first layer of the network includes 13 times 6 weights plus 6 threshold values. The last layer of the network includes 6 weights plus 1 threshold value per output, which for an output results in a total of 91 constants. The experience or knowledge of the perceptron is found in these 91 constants, and an improved knowledge is obtained by adjusting said constants.

The network is trained by the constants being subjected to an adjustment in such a manner that the output and the desired output approach each another. As a measurement of how well the network is trained, the squares are used which derive from the differences between the output and the desired output summed of all the sets of data and divided by the number of sets of data minus 1:

$$E = \frac{1}{N\text{-}1}\ (T_\mu\text{-}O_\mu)^2$$

Previously, the size was not weighted with the number. Such a procedure has, however, been followed here in order to provide an estimate of the error E which is independent of the size of the set of data. As the sets of data do not change during the training of the perceptron, all the input data can be considered constants. Therefore, the error E can be considered as a function of the weight factors and threshold values of the perceptron:

$$E = E(w).$$

The NRF-method can be further simplified by only one variable being changed at a time, i.e. all the weight factors and threshold values apart from one are kept constant. Now, it is desired to minimize the error E by only changing the above single variable. At the minimum of a function, the derived of the function is zero. Zero points of a function $\phi(x)$ of a variable x are found by iteration by means of the Newton-Raphson-method based on the formula

$$x_2 = x_1 - \frac{\Phi(x_1)}{\Phi'(x_1)} \approx x_1 - \frac{\Phi(x_1)(x_1\text{-}x_0)}{\Phi(x_1)\text{-}\Phi(x_0)}$$

where $x_1$ represents the old x-value and $x_2$ represents the new x-value

In this case the function $\phi$ is the derived value of the error E(x), the error E(x) being considered as a function of a variable x, which results in

$$\Phi = \frac{dE(x)}{dx} \approx \frac{E(x_1)\text{-}E(x_0)}{\Delta x}$$

By combining the above expressions, the following difference formula is obtained

$$x_2 = x_1 - \frac{(E(x_1+\Delta x) - E(x_1-\Delta x))\Delta x}{2(E(x_1+\Delta x) - 2E(x_1) + E(x_1-\Delta x))}$$

Based on this expression, a new x- value ($x_2$) can be calculated for the independent variable x, said new value involving a smaller error compared to the above error ($x_1$). This method is, however, encumbered with the draw-back that 3 function values must be calculated each time.

The described method of minimizing the error E is performed for each iteration for all weights and threshold values in the network. In the network for earth type correction, the weight factors and threshold values are updated immediately after a calculation of a new value. Alternatively, the same set of weight factors and threshold values can be updated after each iteration. Such a procedure would, however, require a large memory as both new and old values for weight factors and threshold values should be stored in said memory until an updating is performed. In return, the number of calculations would be reduced to approximately 2/3.

As the weight factors are changed towards the zero points of the derived value of the error, no security exists that the change does not result in a movement towards a maximum instead of towards a minimum. The method is, however, extremely reliable. An iteration involves a number of calculations exceeding many times the number of calculations involved in an epoch by the error back propagation-method.In return, each epoch is far more efficient.

The method used is decisive for the speed of the training. Some methods are so "careful" that the "movement" through the "knowledge area" stops in the first local minimum. Then, it is possible to "shake the bag" slightly in order to proceed towards a lower minimum. In connection with the NRF-method, a stochastic updating rule is inserted with the result that not all weight factors and threshold values are included in each epoch. As a result, the passing of local minima has been facilitated.

It cannot be concluded that the network is injudicious because the training is slowly performed, because the method chosen determines the speed of the training.

When the error on the output has been minimized, further possibilities of improving the network apply. It can be too complex for the task in question, and accordingly an advantage is found in subjecting each connection to a "saliency" test. Saliency for each connection is calculated by:

$$S = \frac{1}{2} \times \frac{\partial^2 E}{\partial w^2} w^2$$

The connections involving the lowest saliency-values are omitted, and the network is subjected to a new training. This process continues until the network is no longer improved and perhaps even become poorer. Then an optimal "brain damage" is considered to be performed.

Two different jobs have been performed. One job is a pure earth type correction, i.e. the job for the network is to find the original integral counting number on the basis of a normalized spectrum. The other job is to determine both the original integral counting number and the integral counting number for the added amount of potassium.

The program converts the desired output in order to obtain a suitable output interval. The conversion involves a dividing by 100,000 and subsequently a deduction of 0.4. In this manner, all the outputs are close to 0, which is necessary for allowing a calculation of the output by means of the sigmoid-function. A measurement of the capacity of the network to perform earth type correction is then

$$Error = \frac{1}{N-1} (T_\mu - O_\mu)^2$$

The square root of this value is not a variance, but when the average number of the set of data is scaled by 1/100,000, the ratio of the error to the scaled average number can be compared with a relative variance. Below the designation "relative variance" is used.

When a network with six hidden neurons and an output-neuron is programmed for earth type correction for all 104 sets of data, a relative variance of 6.3% is obtained. When the same set of data is used on samples from an agricultural area at a time, the following results are obtained:

| | Deviation % of average | Variance % of average | Deviation % of average | Variance % of average |
|---|---|---|---|---|
| Test group | Training set | Training set | Reference set | Reference set |
| N | 2.58 | 5.56 | 2.79 | 5.86 |
| S | -0.59 | 8.14 | -0.58 | 4.65 |
| O | 1.01 | 6.31 | -0.80 | 8.74 |
| G | 1.94 | 5.73 | -1.07 | 7.77 |
| All | 0.61 | 6.47 | 1.21 | 6.34 |

"The training sets" are the sets of data which the network has been trained on. "The reference sets" are the sets of data which are used for testing the ability of the network of recognizing the encoded pattern in unknown sets of data.

When the numbers for the N-samples are considered the following results can be concluded:

As far as the training set is concerned, an average earth percentage of 10% would have been calculated to 10.258% due to deviations from the average number for these samples. The variance on determinations of earth percentages in these types of earth would then at 10% be ± 0.556% earth.

After the training of the network, the average variance on the training set is usually negligible. However, the reason why the result is 0.61% is that the network in question has been tested on the sets of data relating to addition of potassium as said sets of data are not completely identical with the sets used during the training.

The generation of sets of data with added potassium has been described in the paragraphs describing "sets of data admixed potassium". The generation of sets of data admixed various concentrations of potassium results in an amount of data being seven times higher. Accordingly, the network with two outputs has been trained first on the O- and the G-samples and subsequently on the N- and the S-samples.

In the Table, the uppermost number refers to the relative variance and the lowermost number to the relative variance. A training on the O- and the G-samples results in the following:

| Potassium | N | S | O | G |
|---|---|---|---|---|
| [%] | S[%] Var. [%] | S[%] Var. [%] | S[%] Var. [%] | S[%] Var. [%] |
| O | 8.43 5.83 | 8.60 1.10 | 7.43 2.85 | 7.12 1.76 |
| 10 | 7.34 4.37 | 8.27 -0.11 | 7.14 1.73 | 6.67 1.09 |
| 20 | 6.55 3.11 | 8.16 -1.52 | 7.08 0.76 | 6.37 0.52 |
| 30 | 6.02 2.01 | 8.19 -2.06 | 7.18 -0.09 | 6.19 0.02 |
| 40 | 5.72 1.04 | 8.32 -2.87 | 7.39 -0.84 | 6.09 -0.42 |
| 50 | 5.59 0.18 | 8.51 -3.58 | 7.66 -1.50 | 6.05 -0.81 |
| 60 | 5.61 -0.59 | 8.74 -4.22 | 7.96 -2.10 | 6.05 -1.15 |
| All | 6.432 2.28 | 8.269 -1.84 | 7.242 0.12 | 6.284 0.14 |

From the lowermost line in the Table it appears that no vigorous increase applies to the relative variance where the potassium components differ in the spectra. The price is, however, that the average variance depends on potassium. It appears for instance from the column of the G-samples that the change from the potassium-free spectrum with a deviation of 1.76 to the spectrum with 60% potassium with a deviation of -1.15%, is a change of almost 3% of the calculated earth number. The 60% potassium have, however, been added on the basis of an earth percentage of 10%. As far as an earth percentage of 1% is concerned, the same change in the content of potassium of the beets would probably result in the same absolute change in the earth percentage, viz. from 1 to 1.3%. It is not surprising that distinct deviations appear from the N- and the S-samples because these samples are unknown to the network.

The percentages for the potassium contents have been calculated so as substantially to reflect the situation where 100% represents the potassium concentration providing the same input to the integral counting number as 10% earth in a load. A calculation of the variance on all samples results in 7.1%.

It should be noted that both the earth quantity and the potassium quantity form part of the calculations of the earth percentage. The variance on the potassium quantity does not appear from the Table, but the ability of the network to predict the concentration of potassium converted into relative variance amounts to approximately 5%. The accumulation law provides a total variance on an earth percentage calculated in this manner of 8.7%.

The capacity of the network to predict the concentration of potassium converted into relative variance is approximately 5%.

After a partial training based on N- and S-samples the following is obtained:

| Potassium | N | S | O | G |
|---|---|---|---|---|
| [%] | S [%] Var. [%] | S [%] Var. [%] | S [%] Var. [%] | S[%] Var. [%] |
| O | 7.54 4.79 | 7.57 1.75 | 9.90 3.84 | 9.04 6.60 |
| 10 | 6.72 3.42 | 7.30 0.51 | 9.57 2.64 | 8.29 5.55 |
| 20 | 6.18 2.24 | 7.25 -0.56 | 9.43 1.60 | 7.72 4.66 |
| 30 | 5.90 1.21 | 7.36 -1.49 | 9.42 -0.69 | 7.29 3.89 |
| 40 | 5.80 0.31 | 7.55 -2.31 | 9.50 -0.10 | 6.98 3.22 |
| 50 | 5.84 -0.50 | 8.81 -3.05 | 9.64 -0.81 | 6.76 2.62 |
| 60 | 5.98 -1.21 | 8.09 -3.70 | 9.81 -1.44 | 6.61 2.10 |
| All | 6.202 1.47 | 7.449 -1.26 | 9.391 0.92 | 7.464 4.09 |

It appears that the numbers for the N- and the S-samples have been considerably improved compared to previously. When the network is trained on all samples, the good properties from both Tables can to a certain degree be combined.

Neural networks are suited for non-linear tasks due to a non-linearity built therein. Accordingly, the earth type correction does not necessarily require a neural network because the task is of a linear character, but the use of these networks renders it possible to solve more tasks in one operation. In addition, the network need only be provided with examples in order to be trained for the task to be solved. Such tasks require much calculating power. Once the computer program has been written, it is, however, relatively easy to train a network rather than analyzing and setting up empiric formulae and subsequently encoding said data in a program.

Here attempts have been made at training a network to perform earth type correction on pure earth spectra and spectra with a varying concentrations of potassium, respectively.

For the evaluation of the capacities of the network to calculate a size comparable with a variance relative to the average number, i.e. analogous with relative variance. For the $y$-spectra measured on pure earth the total set of data achieves a value corresponding to a relative variance of 6.3%.

When various amounts of potassium is added, i.e. from 0% to 60% of the integral counting number, the corresponding result is 7.1%. The greatest disadvantage is found in the fact that the calculated earth percentage can be erroneous by up to approximately ±5% of the earth percentage in connection with 10% of earth. When the concentration of potassium is 0%, a too low contents of earth is predicted, and when said concentration of potassium amounts to 60% of the integral counting number a too high contents of earth is predicted.

After the conversion into relative variance, the variance on the prediction of potassium in the spectrum is 5% with the result that the relative variance on one earth percentage calculated in this manner should be 8.7%.

Below, a set of measuring data are analyzed by means of the conventional methods with and without humus correction and by means of the method according to the invention, where a neural network is used.

Here the results achieved by means of the previously used calculating methods are as follows:

| | | Without humus correct. | | With humus correction | |
|---|---|---|---|---|---|
| Earth [%] | Number | Ave.. [%] | Var. [%] | Ave., [%] | Var. [%] |
| 0 | 20 | 0.36 | 0.38 | 0.44 | 0,48 |
| 5 | 16 | 5.16 | 1.11 | 6.07 | 1.07 |
| 10 | 20 | 10.06 | 2.13 | 11.67 | 1.97 |

(continued)

| | | Without humus correct. | | With humus correction | |
|---|---|---|---|---|---|
| Earth [%] | Number | Ave.. [%] | Var. [%] | Ave., [%] | Var. [%] |
| 15 | 20 | 13.66 | 2.30 | 16.25 | 2.77 |
| 20 | 20 | 17.36 | 3.26 | 21.19 | 4.33 |

The results of the Table are good in view of the fact that an uneven distribution of earth in the load increases the variance. The method without humus correction is encumbered with problems at earth percentages of 15 and 20, where the average values are too low. After the introduction of generally speaking, the average values are, generally speaking, too high. The basic element of this calculating method is the square of the potassium window divided by a window about 260 keV (Actinium-228 and Radon-226). A calculation being so rigid are encumbered with problems due to the fact that two types of humus earth are contained in the data material.

The results of neural networks appear from the following Table:

| Earth [%] | Number | Average [%] | Variance [%] |
|---|---|---|---|
| 0 | 20 | 0.05 | 0.35 |
| 5 | 12 | 5.05 | 1.26 |
| 10 | 20 | 10.28 | 1.59 |
| 15 | 20 | 14.89 | 2.11 |
| 20 | 20 | 19.67 | 2.48 |

It appears that the average values are in the correct positions. Compared to the calculations with and without humus correction, a considerable reduction is furthermore found in the variance.

## Claims

1. A method of detecting the contents of earth in agricultural crops, such as beets, by measuring the natural $y$-radiation of the earth, the counting number of the measured $y$-radiation being compensated for differences in the types of earth by an analysis of the $y$-spectrum, **characterised** in that the $y$-spectrum is analyzed by means of a neural network with a high number of inputs, a signal representing the counting number of a separate energy range for the $y$-radiation being fed to each input, where the neural network has been trained by means of known references.

2. A method as claimed in claim 1, **characterised** by the neural network comprising 14 inputs, 13 inputs for the introduction of the counting number in 13 different windows and an input for the introduction of the integral counting number.

3. A method as claimed in claim 1 or 2, **characterised** by the neural network comprising four layers.

4. A method as claimed in one or more of the preceding claims, **characterised** in that the neurons not being used are eliminated during the training of the neural network by means of known references.

5. A method as claimed in one or more of the preceding claims, **characterised** by compensating for background radiation.

## Patentansprüche

1. Verfahren zum Nachweis des Gehalts an Erde in Landbauprodukten, wie Rüben, durch Messen der natürlichen $\gamma$-Strahlung der Erde, wobei die Zählrate der gemessenen $\gamma$-Strahlung auf Unterschiede in den Erdtypen hin durch Analyse des $\gamma$-Spektrums kompensiert wird, dadurch gekennzeichnet, daß das $\gamma$-Spektrum mittels eines neuronalen Netzwerks mit einer hohen Anzahl von Eingängen gemessen wird, wobei jedem Eingang ein Signal zugeführt wird, das die Zählrate eines getrennten Energiebereichs für die γ -Strahlung wiedergibt, und das neuronale Netzwerk mittels bekannter Referenzen trainiert wurde.

2. Verfahren nach Anspruch 1, dadurch gekennzeichnet, daß das neuronale Netzwerk 14 Eingänge umfaßt, nämlich 13 Eingänge für die Eingabe der Zählrate in 13 verschiedenen Fenstern sowie einen Eingang für die Eingabe der Integralzählrate.

3. Verfahren nach Anspruch 1 oder 2, dadurch gekennzeichnet, daß das neuronale Netzwerk vier Schichten umfaßt.

4. Verfahren nach einem oder mehreren der vorstehenden Ansprüche, dadurch gekennzeichnet, daß die nicht benutzten Neuronen während des Trainings des neuronalen Netzwerks mittels bekannter Referenzen beseitigt werden.

5. Verfahren nach einem oder mehreren der vorstehenden Ansprüche, dadurch gekennzeichnet, daß die Untergrundstrahlung kompensiert wird.

**Revendications**

1. Un procédé de détection de la teneur en terre de produits de récolte agricole, tels que des betteraves, en mesurant le rayonnement γ naturel de la terre, le nombre de comptage du rayonnement γ mesuré étant compensé pour les différences entre les types de terre par une analyse du spectre γ, caractérisé en ce que le spectre γ est analysé au moyen d'n réseau neuronal avec un grand nombre d'entrées, un signal représentant le nombre de comptage d'une bande d'énergie séparée pour le rayonnement γ étant fourni à chaque entrée, le réseau neuronal ayant été entraîné au moyen de références connues.

2. Un procédé selon la revendication 1, caractérisé en ce que le réseau neuronal comprend 14 entrées, 13 entrées pour l'introduction du nombre de comptage dans 13 fenêtres différentes et une entrée pour l'introduction du nombre de comptage entier.

3. Un procédé selon la revendication 1 ou 2, caractérisé en ce que le réseau neuronal comprend quatre niveaux.

4. Un procédé selon au moins l'une des revendications précédentes, caractérisé en ce que les neurones non utilisés sont éliminés pendant l'entraînement du réseau neuronal au moyen des références connues.

5. Un procédé selon au moins l'une des revendications précédentes, caractérisé en ce que l'on compense le rayonnement naturel.

FIG. 1

FIG. 2

FIG. 3

FIG. 4

Correlations between output of a neural
network and the dry matter in earth

NN -output

Correlation:

NN-trn.: 0.76
NN-ref.: 0.74

Dry matter [%]

FIG. 5

■ NN Tr.

+ NN Rf.

NN-determination of flint% in earth samples
NN-input:7 set windows

FIG. 6

EP 0 749 575 B1